# EUROPEAN PATENT SPECIFICATION

(11) **EP 4 138 994 B1**
(45) Date of publication and mention of the grant of the patent: **22.10.2025**
(21) Application number: 21793547.7
(22) Date of filing: 21.04.2021
(51) Int. Cl.: A61N 1/39, A61N 1/04, G16H 20/30, G16H 40/20, G16H 40/63

(54) **RESUSCITATION DEVICE**
WIEDERBELEBUNGSVORRICHTUNG
DISPOSITIF DE RÉANIMATION

(30) Priority: 23.04.2020 US 202063014127 P
(43) Date of publication of application: 01.03.2023
(73) Proprietor: Joltz LLC, Passaic, New Jersey 07055 (US)
(72) Inventor: ROSEMAN, Yehuda, 99543 Bet Shemesh (IL); MASSE, Daniel, Windham, New Hampshire 03087 (US); WELCHES, Shaun, TOWNSEND, Massachusetts 01469 (US); KISHINEVSKY, Michael, Andover, Massachusetts 01845 (US); MCGINN, Sam, Billerica, Massachusetts 01821 (US); ROSENBLUM, Jonathan, 99543 BET SHEMESH (IL)
(74) Representative: Schulz Junghans Patentanwälte PartGmbB
(86) International application number: PCT/IL2021/050456
(87) International publication number: WO 2021/214768

(56) References cited:
- WO-A1-2019/070516
- US-A1- 2018 169 426
- US-A1- 2019 282 822
- US-A1- 2020 108 261
- US-B2- 9 067 080

## Description

### FIELD OF INVENTION

The present disclosure generally relates to resuscitation devices.

### BACKGROUND

A primary task of the heart is to pump oxygenated, nutrient-rich blood throughout the body. Electrical impulses generated by a portion of the heart regulate the pumping cycle. When the electrical impulses follow a regular and consistent pattern, the heart functions normally and the pumping of blood is optimized. When the electrical impulses of the heart are disrupted (i.e., cardiac arrhythmia), sudden cardiac arrest may result, which inhibits the circulation of blood. As a result, the brain and other critical organs are deprived of nutrients and oxygen. A person experiencing sudden cardiac arrest may suddenly lose consciousness and die shortly thereafter if left untreated.

A well known and effective treatment for sudden cardiac arrest or arrhythmia is defibrillation. Defibrillation involves passing a current through the person to shock the heart back into a normal rhythm. There are a wide variety of resuscitation devices. For example, implantable cardioverter-resuscitation devices ("ICD") involve surgically implanting wire coils and a generator device within a person. ICDs are typically for people at high risk for a cardiac arrhythmia. When a cardiac arrhythmia is detected, a current is automatically passed through the heart of the user with little or no intervention by a third party.

Another, more common type of resuscitation device is the automated external resuscitation device ("AED"). Rather than being implanted, the AED is an external device used by a third party to resuscitate a person who has suffered from sudden cardiac arrest. A conventional AED includes a base unit and two pads connected to the base unit using electrical cables. Sometimes electrodes with handles are used instead of the pads.

A typical protocol for using the AED on a subject who has suffered from sudden cardiac arrest (the "patient") is as followed. The patient is initially placed on the floor. Clothing on the subject is removed to reveal the chest. The AED pads are applied to appropriate locations on the chest. The electrical system within the base unit generates a high voltage between the two pads, which delivers an electrical shock to the subject. Ideally, the shock restores a normal cardiac rhythm, and in some cases, multiple shocks are required.

Particularly, US2018/169426A1 relates to a device, and software and methodology associated with a portable Automated External Defibrillator ("AED"). The portable AED works with a mobile device and software, and includes two or more cardiac pads, a battery pack, and specialized capacitor. When connected to a patient in cardiac arrest, the AED contacts Emergency Medical Services, and records patient information to be transmitted for evaluation by medical providers.

Furthermore, US2020/108261A1 discloses an automated external defibrillator that can be powered by a mobile communication device such as a smart cellular phone or a tablet computer.

### SUMMARY

The following embodiments and aspects thereof are described and illustrated in conjunction with systems, tools and methods which are meant to be exemplary and illustrative, not limiting in scope.

There is provided, in accordance with the invention, a resuscitation device, including an electric pulse generator configured to generate an electric pulse that is administered to a subject, electrodes operative to administer the electric pulse to the subject, one or more sensors configured to measure vital signs of the subject, one or more processing units configured to monitor the vital signs measured by the one or more sensors, determine the housing and electrodes are properly placed on the subject according to the monitoring of the vital signs, determine what treatment has to be administered to the subject, generate notification instructing the treatment to be administered to the subject, and providing real-time, continuous feedback of treatment provided and condition of the subject.

The resuscitation device is further configured to recognize that the mobile device is within a predetermined distance from the resuscitation device, receive authentication information from the mobile device, determine the authentication information matches a stored authentication information, grant the mobile communication access to communicate with the resuscitation device.

The resuscitation device is further configured to determining a plurality of mobile device have authority to access the resuscitation device, allow access to the mobile device having a highest priority according to a priority hierarchy.

In some embodiments, the electric pulse generator includes an electrical circuit for generating an electric pulse, said electric circuit includes a controller, a switch, an inductor, a charging circuit, and a capacitor array electrically connected to the charging circuit and adapted to store energy supplied by the charging circuit, where the controller is adapted to control the switch to cause energy to be stored in the inductor and discharged to the charging circuit.

In some embodiments, the controller is a controller and gate driver chip, the inductor is a low-profile boost inductor, and the charging circuit is a plurality of interconnected charging capacitors and diodes.

In some embodiments, the controller chip, the switch, and charging capacitor are surface mount components.

In some embodiments, the circuit includes an electric pulse generator for a low profile AED.

In some embodiments, the capacitor array includes a plurality of interconnected discrete energy-storing capacitors capable of storing up to a predetermined amount of bi-phasic electric pulse energy.

In some embodiments, the capacitor array has a total capacitance of at least 100 microfarads and 1.5 kilovolts to provide at least 150 joules of energy storage.

In some embodiments, the resuscitation device further includes a communication unit configured to communicate with a mobile device, the mobile device configured to present the user with user interface and instructional content, receive user commands for administering the electric pulse to the subject, transmit the user commands to the communication unit.

In some embodiments, the resuscitation device further includes a housing, a case operative to store the housing and the electrodes.

In some embodiments, the resuscitation device further includes a case, an activation switch, a sensor case configured to detect when the case is opened, where the processor is configured to switch from a sleep mode to a hibernation mode when the case sensor detects the case is opened, switch to an active mode when the activation switch is engaged.

There is further provided in accordance with an embodiment, a system including a resuscitation device configured to measure and monitor vital signs of a subject, administer an electric pulse to the subject, provide instruction to a treatment provider of the treatment provided to the subject, one or more mobile devices configured to be in wireless communication with the resuscitation device, the v configured to, receive the vital signs, present a user of the mobile device with a user interface configured to, display the vital signs, and obtain commands from the user that are provided to resuscitation device, transmit the commands to the resuscitation device, the resuscitation device executes the commands received from the mobile device.

In some embodiments, the resuscitation device includes one or more sensors configured to measure the vital signs, an electric pulse generator configured to generate the electric pulse, at least two electrodes configured to administer the electric pulse.

In some embodiments, the electric pulse generator includes a capacitor array configured to facilitate storing the electric pulse prior to administration.

In some embodiments, the resuscitation device further includes a tarp operative to facilitate covering the chest of the subject during administration of medical treatment.

There is further provided in accordance with a non-claimed embodiment, a resuscitation device configured to administer an electric pulse to a subject, including electrodes operative to administer an electric pulse to a body of the subject, and an electric pulse generator configured to generate the electric pulse that is administered to via the electrodes to the subject, the electric pulse generator including an electric circuit configured to store an electric charge sufficient to generate the electric pulse.

In some embodiments, the resuscitation device further includes one or more sensors configured to measure vital signs of the subject, one or more processing units configured to monitor the vital signs measured by the one or more sensors, determine the housing and electrodes are properly placed on the subject according to the monitoring of the vital signs, determine what treatment has to be administered to the subject, generate notification instructing the treatment to be administered to the subject, providing real-time, continuous feedback of treatment provided and condition of the subject.

There is further provided in accordance with another non-claimed embodiment, an electrical circuit for generating an electric pulse, including a controller, a switch, an inductor, a charging circuit, and a capacitor array electrically connected to the charging circuit and adapted to store energy supplied by the charging circuit, where the controller is adapted to control the switch to cause energy to be stored in the inductor and discharged to the charging circuit.

In some embodiments, the controller is a controller and gate driver chip, the inductor is a low-profile boost inductor, and the charging circuit is a plurality of interconnected charging capacitors and diodes.

In some embodiments, the controller chip, the switch, and charging capacitor are surface mount components.

In some embodiments, the circuit includes an electric pulse generator for a low profile AED.

In some embodiments, the capacitor array includes a plurality of interconnected discrete energy-storing capacitors capable of storing up to a predetermined amount of bi-phasic electric pulse energy.

In some embodiments, the capacitor array has a total capacitance of at least 100 microfarads and 1.5 kilovolts to provide at least 150 joules of energy storage.

In addition to the exemplary aspects and embodiments described above, further aspects and embodiments will become apparent by reference to the figures and by study of the following detailed description.

### BRIEF DESCRIPTION OF THE DRAWINGS

Some non-limiting exemplary embodiments or features of the disclosed subject matter are illustrated in the following drawings.
Fig. 1 shows a resuscitation device connected to a subject for administering an electric pulse, according to certain exemplary embodiments;
Figs. 2A-2C schematically illustrates the resuscitation device in various configurations, according to certain exemplary embodiments;
Fig. 3 schematically illustrates a system for operation of the resuscitation device, according to certain exemplary embodiments;
Fig. 4 schematically illustrates an electrical circuit of the resuscitation device of Fig. 3 configured for charging the electric pulse generator, according to certain exemplary embodiments;
Fig. 5 schematically illustrates a capacitor array of the electrical circuit of Fig. 4, according to certain exemplary embodiments;
Fig. 6 outlines operations of a method for connecting the resuscitation device with a mobile device, according to certain embodiments; and,
Fig. 7 outlines operations of a method for administering the electric pulse to the subject, according to exemplary embodiments; and,
Fig. 8 outlines operations of a method for activating the resuscitation device, according to certain exemplary embodiments.

Identical, duplicate, equivalent or similar structures, elements, or parts that appear in one or more drawings are generally labeled with the same reference numeral, optionally with an additional letter or letters to distinguish between similar entities or variants of entities, and may not be repeatedly labeled and/or described.

Dimensions of components and features shown in the figures are chosen for convenience or clarity of presentation and are not necessarily shown to scale or true perspective. For convenience or clarity, some elements or structures are not shown or shown only partially and/or with different perspective or from different point of views. References to previously presented elements are implied without necessarily further citing the drawing or description in which they appear.

### DETAILED DESCRIPTION

Disclosed herein is a system and method, not part of the claimed invention as such but useful for the general understanding of the invention, for administering resuscitation, according to certain exemplary embodiments.

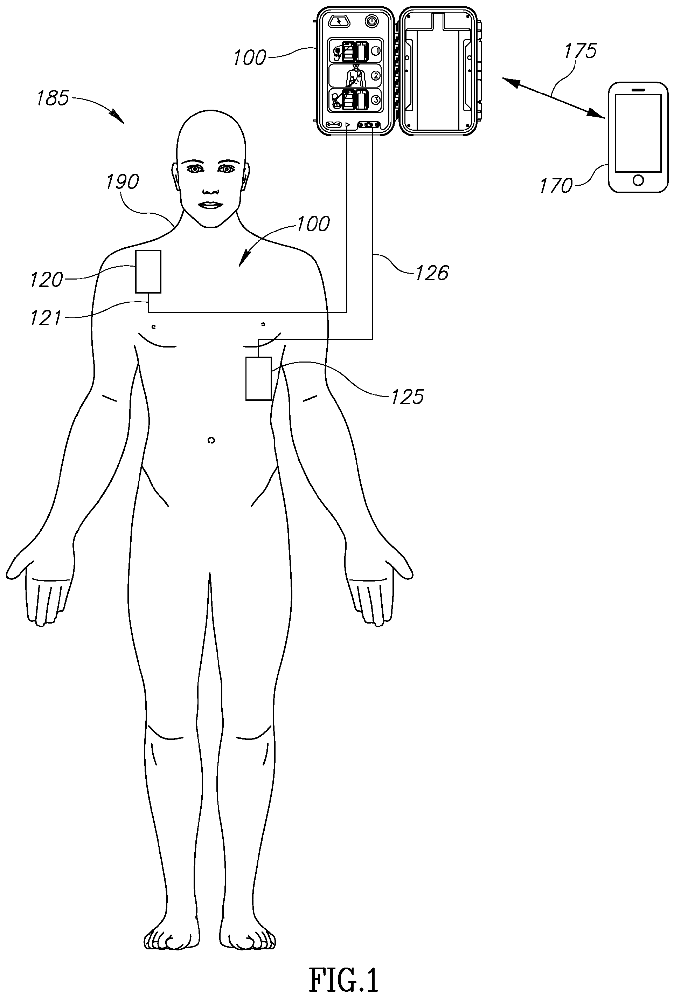

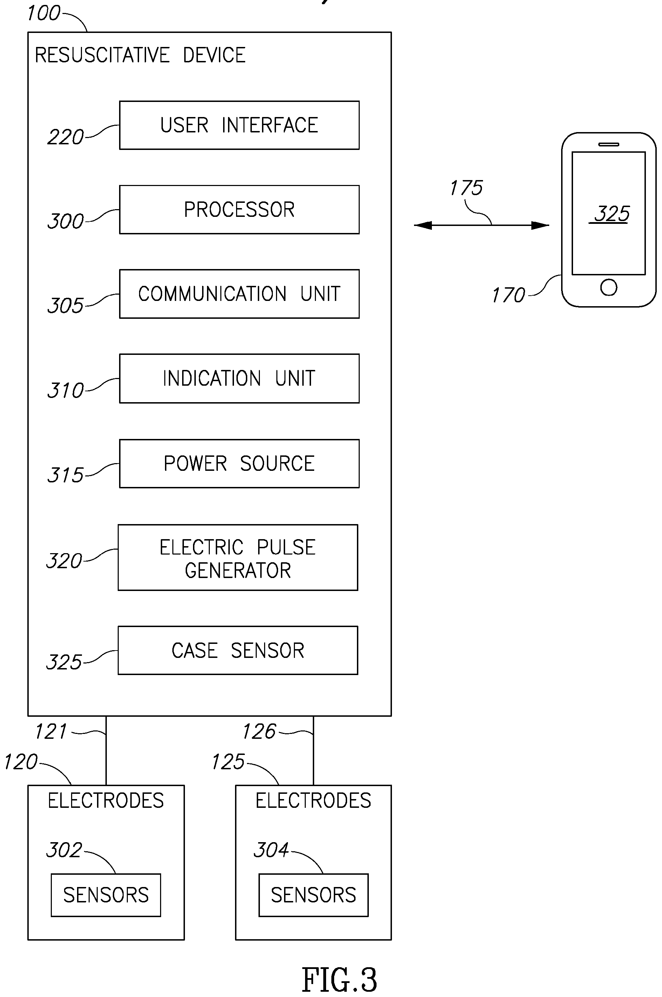

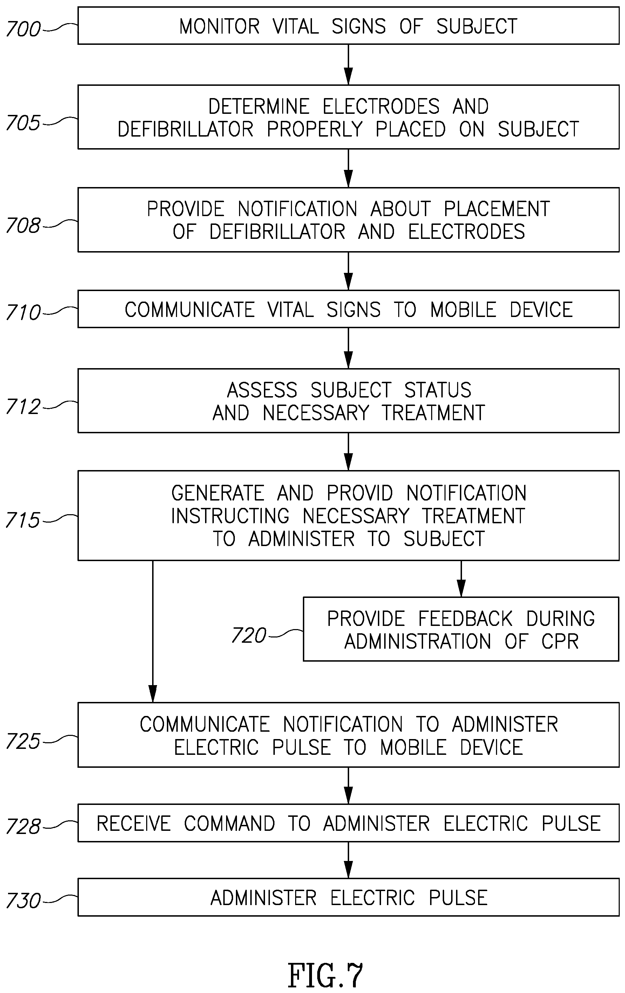

Fig. 1 schematically illustrates a resuscitation device 100 for administering an electric pulse to a subject 185, according to certain embodiments. Resuscitation device 100 includes electrodes 120, 125 that are positioned on a body 190 of subject 185 during an arrythmia incident. Resuscitation device 100 is configured to pass electric pulses of a selected shape and size through body 190 via electrodes 120 and 125. Electrodes 120 and 125 are connected by leads 121 and 126, respectively, to resuscitation device 100. In some embodiments, electrodes 120, 125 are configured to measure vital signs when attached to body 190 thereby facilitating a treatment provider (not shown) to monitor a condition of subject 185 and determining the necessary treatment required and whether an electric pulse needs to be administered, as further described in conjunction with Fig. 3. Resuscitation device 100 is configured to communicate, represented by arrow 175, with a mobile device 170 that is operated by the treatment provider as described in conjunction with Figs 3 and 7.

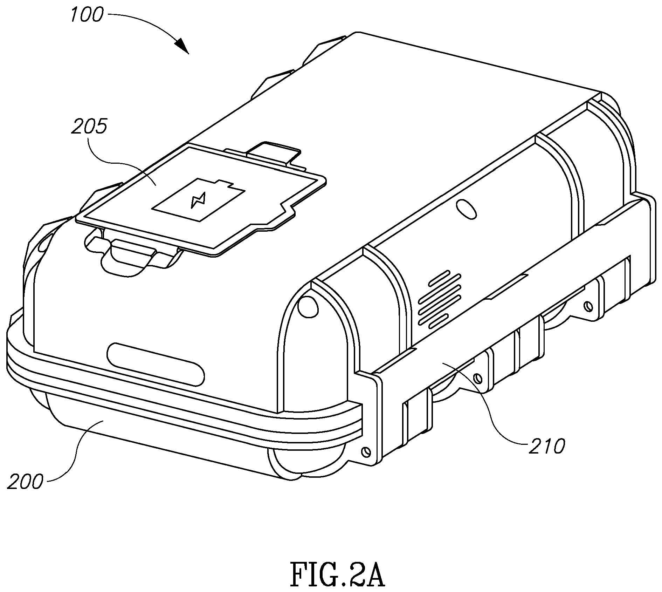

Fig. 2A-2C schematically illustrates resuscitation device 100, according to certain exemplary embodiments. Fig. 2A shows resuscitation device 100 having a housing 200, according to certain embodiments. Housing 200 is configured to provide convenient carrying and storage of resuscitation device 100 when it is not in use. In some embodiments, housing 200 includes a power access port 205 configured to provide access to a power source 315 (Fig. 3), for example, for replacement of a battery or to connect resuscitation device to an external power source such as an electrical socket. Housing 200 include a clip 210 configured to close housing 200 and prevent it from opening when resuscitation device 100 is not in use.

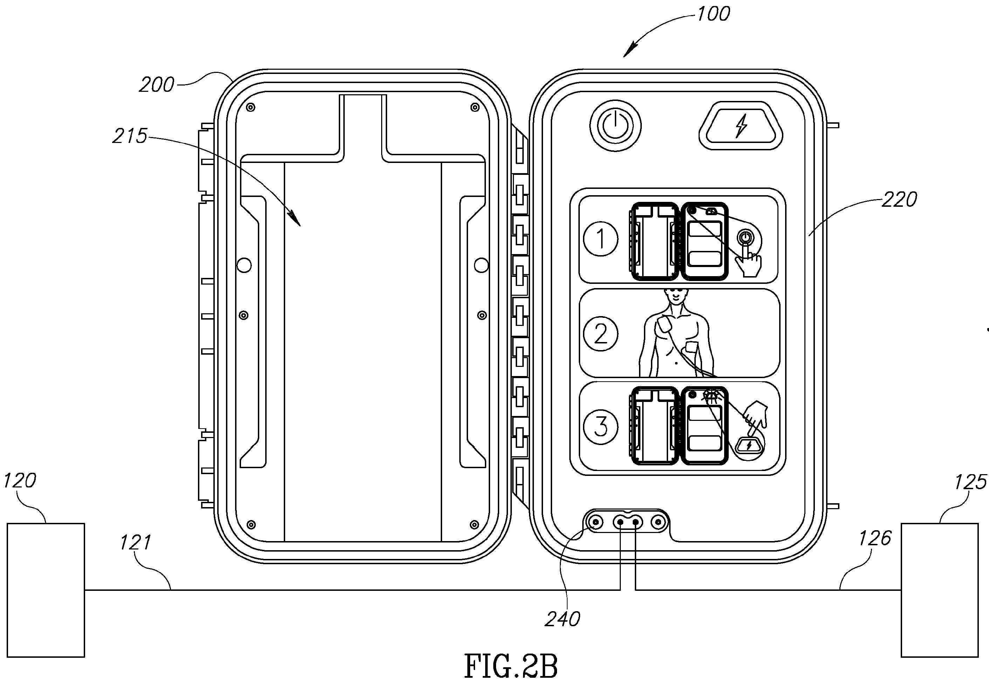

Fig. 2B shows an open configuration of resuscitation device 100, according to certain exemplary embodiments. Prior to operation of resuscitation device 100, housing is opened to expose an internal storage area, referenced as 215, for storing electrodes 120, 125, leads 121, 126, a user interface 220, or the like. Internal storage area 215 includes a connector 240 for connecting leads 125, 126 to resuscitation device 100.

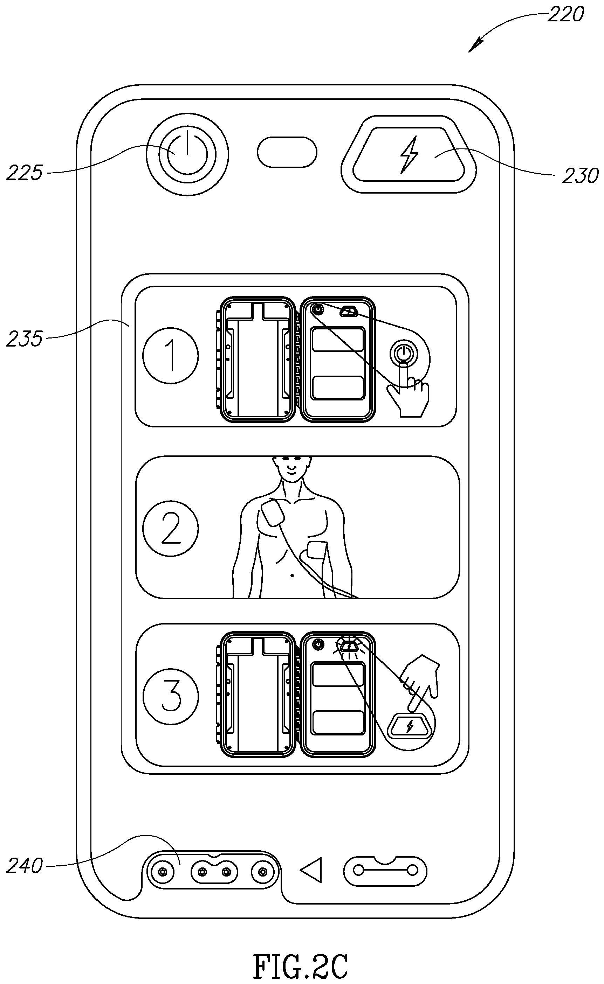

Fig. 2C shows user interface 220, according to certain embodiments. User interface 220 includes a power button 225, a pulse administration button 230 and an instruction interface 235 to provide the treatment provider with instructions for operating the resuscitation device 100. Instruction interface 235 can include pictures, diagrams or the like that show the operating procedures for resuscitation device 100.

**In** some embodiments, resuscitation device 100 is configured to toggle in and out of a low power mode, via opening of housing 200 or engagement of power button 225. When resuscitation device 100 is powered up for the first time, it performs a series of power tests after which it waits for electrodes 120, 121 to be attached to subject 190.

Fig. 3 schematically illustrates components of resuscitation device 100, according to certain exemplary embodiments. Resuscitation device 100 includes one or more processors 300, a communication unit 305, an indication unit 310, power source 315 and an electric pulse generator 320. Electrodes 120, 125 include one or more sensors referenced generally 302, 304, configured to measure vital signs of subject 185 (Fig. 1) when electrodes 120, 125 are attached to body 190 (Fig. 1). In some embodiments, the vital signs can include heart rate, blood pressure, breathing rate, or the like.

Communication unit 305 can be configured to connect and communicate, represented by arrow 175, with mobile device 170. In some embodiments, mobile device 170 is operated by an authorized treatment provider. Communication unit 305 can be configured to continuously transmit the vital signs in real-time to mobile device 170 and to receive commands from mobile device 170, for example, a command to administer an electric pulse to the subject 190.

Mobile device 170 can include an application executed by mobile device 170 to present a mobile user interface 325 configured to display the vital signs received from resuscitation device 100. Mobile user interface 325 can include an input through which the treatment provider can input a command to administer an electric pulse to the subject. In some embodiments, mobile user interface 325 can include a display that shows a tutorial video provided by the application that assists the treatment provider with operating resuscitation device 100. In some embodiments, mobile device 170 can provide an audio tutorial to assist with operating resuscitation device 100.

Indication unit 310 can be configured to provide audio and visual instruction for placement of electrodes 120, 125 onto subject 185 and for administration of an electric pulse to subject 185. Electric pulse generator 320 generates the electric pulse that is administered to subject 185 through electrodes 120, 125.

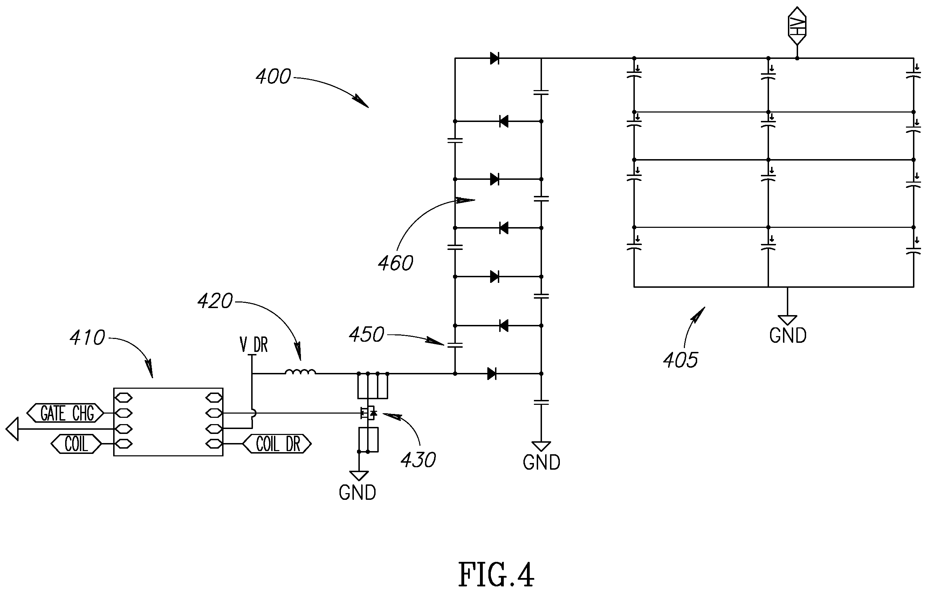

Processor 300 is configured to operate resuscitation device and to regulate communication with mobile device 170 as described in conjunction with Figs. 4-5.

Electric pulse generator 320 is preferably configured to include a capacitor flat pack array 405 (Fig. 4) to replace a capacitor for generating the electric pulse. Reference is now made to Fig. 4., schematically illustrating an electric circuit 600 of electric pulse generator 320, according to certain embodiments.

In some embodiments, resuscitation device 100 can also includes a case sensor 325 configured to detect when case 200 (Fig. 2) is opened. Resuscitation device 100 can remain in a hibernation mode while case 200 remains closed. In such embodiments, when case 200 is opened, case sensor 325 detects the opening of case 200 which switches resuscitation device 100 from the hibernation mode to a sleep mode. During sleep mode, resuscitation device 100 can execute periodic self-tests and wait to detect a microcurrent or other signal from electrodes 120, 125. Upon detection of a microcurrent or any other predetermined condition, resuscitation device 100 can switch to a full operation mode during which an electric pulse can be administered. In some embodiments, electric pulse generator 320 automatically initiates the charging of an electric charge for use as an electric pulse once resuscitation device 100 switches to sleep mode.

Reference is made to Fig. 4, schematically illustrating an exemplary electrical circuit 400 of electric pulse generator 320 configured for generating the electric pulse to be administered to subject 185, according to certain exemplary embodiments. Electrical circuit 400 schematically represents a low-profile charging circuit including a controller and gate driver chip 410, a low-profile boost inductor 420, a semiconductor switch 430, a plurality of charging capacitors 450, a plurality of in-parallel, alternating direction, charging diodes 460, and an output energy storage capacitor array 405. Electrical circuit 400 is configured to enable a treatment charge to be accumulated and stored therein, facilitating a bi-phasic electric pulse discharge to subject 185 (Fig. 1) during defibrillation thereby returning subject 185 to sinus rhythm. Once discharged, electric circuit 400 may recharge output energy storage capacitor array 405 for a subsequent electric pulse discharge that can be delivered by electric pulse generator 320.

This circuit design enables a "very low profile" charging topology for any electric pulse generator requiring such a low profile form factor, whether used in a portable AED or other applications. Using a multiplicity of diodes 460 and charging capacitors 450 and replacing the transformer typically found in the pulse generators of many AED's with boost inductor 420 for the charging circuit is advantageous because it allows for lower overall circuit height (low physical profile). One primary reason is that inductor 420, while similar in construction to a transformer in that both entail windings, needs to store less energy in this circuit design than in other designs and consists of only a single winding, whereas a larger transformer requires at least two windings. This lower profile component enables a reduced height for enclosing charging circuit 400. In some embodiments, other components in electrical circuit 400 are preferably very low profile "surface mount' components.

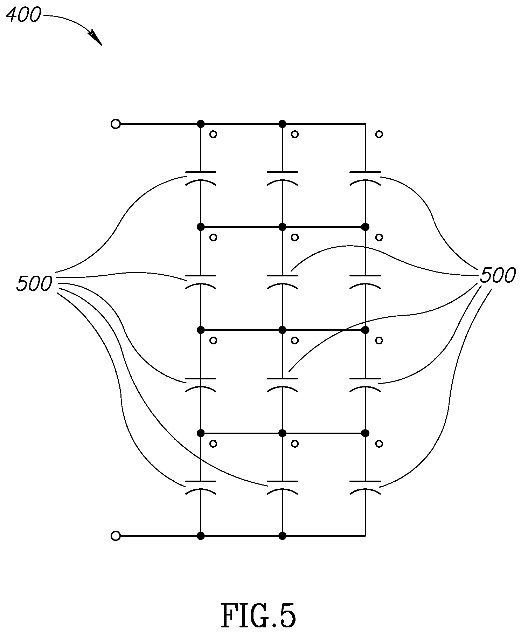

Fig. 5 schematically illustrates in greater detail capacitor array 405 of electrical circuit 400, according to certain exemplary embodiments. Capacitor array 405 is configured to store the necessary bi-phasic electric pulse energy amongst a plurality of series and parallel connected discrete capacitors 500. In some embodiments, capacitor array 405 stores a bi-phasic electric pulse energy of 150 Joules. While a single capacitor could meet all electrical requirements for electric pulse generator 320, capacitor array 405 facilitates a more flexible packaging of the overall electric pulse generator 320 than a single capacitor, thereby further enabling a reduction in the size dimension of resuscitation device 100, enabling an extremely compact, portable design.

Turning back to Fig. 4, combining these space saving designs in circuit 400 may enable the form factor of electric pulse generator 320 and in turn, resuscitation device 100 to be very compact. For example, in some embodiments, resuscitation device 100 designed with electric charging circuit 400 can have a height within a range of 1-1.5 inches, a length within a range of 6-6.50 inches and a width within a range of 3.6-3.65 inches.

In operation, controller chip 410 controls switch 430 to turn on and thereby drive current through boost inductor 420 resulting in energy stored in the magnetic field of inductor 420. Controller 410 thereafter turns off switch 420, thereby allowing inductor 420 to discharge through the multiplicity of charging diodes 460 and into charging capacitors 450. Controller chip 410 monitors output voltage which accumulates in storage capacitor array 405 and adjusts the duty cycle of switch 430 to regulate the output voltage and thus the energy stored in the capacitor array 405.

Capacitor array 405 includes an array of a predetermined number of capacitors 500, and in the present embodiment 12 capacitors, configured to provide requisite energy accumulation and storage of the electric pulse energy. For example, capacitor array 405 can include a number of capacitors 500 within a range of 9-16. In some applications, the number of storage capacitors in array 500 may be as few as 2 and as many as 64 or more. In certain cases, where capacitor array 405 includes twelve capacitors 500 as shown, each capacitor 500 can have a capacitance of 140 Microfarads ("µF") and voltage rating of 425 Volts ("V") each, thereby achieving a total capacitance of 105µF and 1.7 kilovolts ("kV") to provide approximately 150 Joules of energy storage.

Reference is made back to Fig. 3. Mobile device 170 can execute an application that provides a user of mobile device 170, such as a treatment provider, with a user interface 325 that enables the treatment provider to communicate with resuscitation device 100 and monitor the vital signs and condition of subject 185. User interface 325 can present video and audio instructions to the treatment provider facilitating the operation of resuscitation device 100 and to enable treatment provider to provide commands to resuscitation device 100. For example, treatment provider can command resuscitation device 100 to administer an electric pulse through user interface 325. User interface 325 can display an instructional video presentation providing step-by-step instructions to the user to correctly operate resuscitation device 100, for example, a video and audio instructional that after each instructional step gives the provider time to perform the step. Resuscitation device 100 can provide mobile device 100 with real-time feedback about performance of the step and user interface 178 can provide the treatment provider with additional instructional material, repeat the instructional step or continue to the next step according to the performance of the treatment provider.

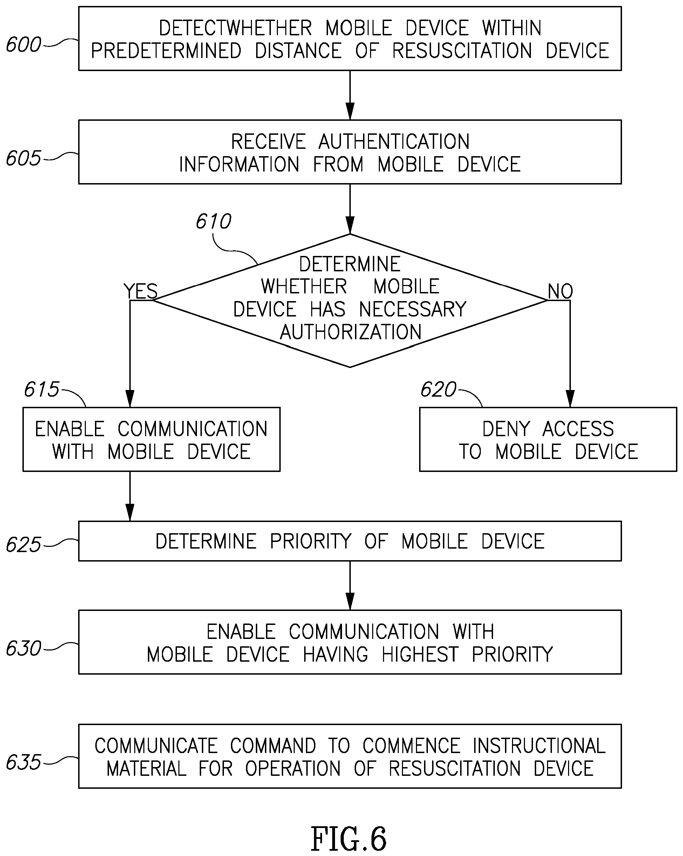

Reference is now made to Fig. 6, outlining operations executed by processor 300 (Fig. 3) to associate resuscitation device 100 (Fig. 1) with mobile device 170 (Fig. 1), according to certain embodiments. When emergency services are notified of a medical emergency requiring operation of resuscitation device 100, the emergency services can notify a treatment provider of the incident and the treatment provider is dispatched to the event. The treatment provider can be dispatched according to vicinity to the resuscitation device 100, which can be determined by registration of resuscitation device 100 with a location-based crowdsourcing platform or, in conjunction with any conventional geo-locating technology, with medical services within a predetermined area, such as a city, district, state, and/or the like.

When the treatment provider is within a predetermined distance from resuscitation device 100, processor 300 executes operation 600 detecting whether mobile device 170 is within a predetermined distance from resuscitation device 100. Processor 300 detects whether mobile device 170 is within a predetermined distance from resuscitation device 100, for example from pings received from mobile devices near resuscitation device 100. For example, the distance can be within a 1-meter radius from a location of resuscitation device 100.

In operation 605, processor 300 receives authentication information from mobile device 170 from communication unit 305. Communication unit 305 receives the authentication information from mobile device 170 as described in conjunction with Fig. 3.

In operation 610, processor 300 determines whether mobile device 170 has a necessary authorization to enable communication between to resuscitation device 100 and mobile device 170. In some embodiments, processor 300 compares the authentication information received from mobile device 170 with stored authentication information. The stored authentication information can be authentication information that is registered with resuscitation device 100 prior to a medical emergency, for example, during production, first time activation and/or the like. The stored authentication information can include authentication information for all treatment providers within a predetermined area such as a city, district, and/or the like, or can be of predetermined organizations such as the red cross, first aid responders and/or the like. In certain embodiments, registration can be achieved through crowdsourcing platforms.

Where the authentication information matches the stored authentication information, processor executes step 615 enabling communication 175 (Fig. 1) between resuscitation device 100 and mobile device 170. Where the authentication information does not match the stored authentication information mobile device 170, processor 300 executes step 620 denying mobile device 170 access to resuscitation device.

In certain exemplary embodiments, multiple mobile devices may have authentication information that will enable communication with resuscitation device 100. In such cases a hierarchy of authentication can be stored in resuscitation device 100, and processor 300 executes optional step 625 to determine a priority of mobile device 170. According to the priority of mobile device 170, processor 300 prioritizes mobile device 170 over other mobile devices according to the hierarchy of authentication based on the authentication information of mobile device 170 and executes step 430 to enable communication with mobile device 170 with highest priority.

In operation 635, processor 300 initiates outputting of instructional material for operation of resuscitation device 100. Processor 300 provides the command to communication unit 305, which transmits the command to mobile device 170. Upon receipt of the command, mobile device 170 initiates instructional presentation in the application of mobile device 170. The instructional presentation can be in the form of a video and/or audio presentation that guides the user of mobile device 170 to properly position electrodes 120, 125 (Fig. 1) on subject 185 and when to administer an electric pulse to subject 190.

Fig. 7 outlines operations executed by processor 300 (Fig. 3) for administering treatment to the subject, according to exemplary embodiments.

In operation 700, processor 300 monitors vital signs which it receives from sensors 302, 304 (Fig. 3) in electrodes 120, 125 (Fig. 1). In some embodiments, sensors 302, 304 activate once the seal covering electrodes 120, 125 is removed.

In operation 705 processor 300 determines whether electrodes 120, 125 (Fig. 1, 3) have been positioned at designated locations on subject 185 (Fig. 1). Processor 300 determines proper positioning of electrodes 120, 125 according to the vital signs measured by sensors 302, 304 in electrodes 120, 125.

In operation 708, processor 300 generates a notification for mobile device 170 informing the user whether electrodes 120, 125 are positioned at the designated locations. Processor 300 transfers the notification to communication unit 305 which transmits the notification to the mobile device 170. In some embodiments, the notification is accessible via user interface 325 (Fig. 1). The notification can inform the user that electrodes 120, 125 are properly positioned at the designated locations or that they are not properly positioned and must be moved to the designated locations, after which processor 300 executes operations 500, 505 and 508 until electrodes 120, 125 are properly placed.

In operation 710, processor 300 operates communication unit 310 to transmit the vital signs to mobile device 170, which are then be displayed on user interface 325. Processor 300 operates communication unit 305 (Fig. 3) to continuously transmit in real time the vital signs measured by sensors 302, 304 to mobile device 170. Through monitoring of the vital signs transmitted to mobile device, the treatment provider can assess the condition of the subject and determine the best course of treatment.

In operation 712, processor 300 assess the status of the subject 185 and generates a treatment recommendation. Processor 300 analyzes the vital signs thereby making an assessment of the condition of the subject 185. In some embodiments, the analysis is achieved by comparing the vital signs obtained by sensors 302, 304 with and determines the treatment necessary to subject 185. For example, processor 300 can determine subject 185 is suffering from heart arrythmia and therefore an electric pulse must be administered to subject 185.

In operation 715, processor 300 generates a notification instructing the necessary treatment for the subject, which is transmitted by communication device 305 to mobile device 170.

In operation 725, processor 300 generates a pulse administration notification to notify treatment administer to administer an electric pulse to the subject 185. The notification is transmitted by communication unit 305 to mobile device 170 to be displayed on user interface 325 to enable treatment provider with the ability to provide a command to administer the electric pulse.

In operation 730, processor 300 receives a command to administer an electric pulse to the subject 325.

In operation 740, processor 300 administers the electric pulse to the subject 190. Processor 300 operates electric pulse generator 169 to generate an electric pulse that is administered to subject 185 via electrodes 120, 125. After administration of the electric pulse, processor 300 continuously executes operations 710, 712, 715 and operation 725, 730 if necessary.

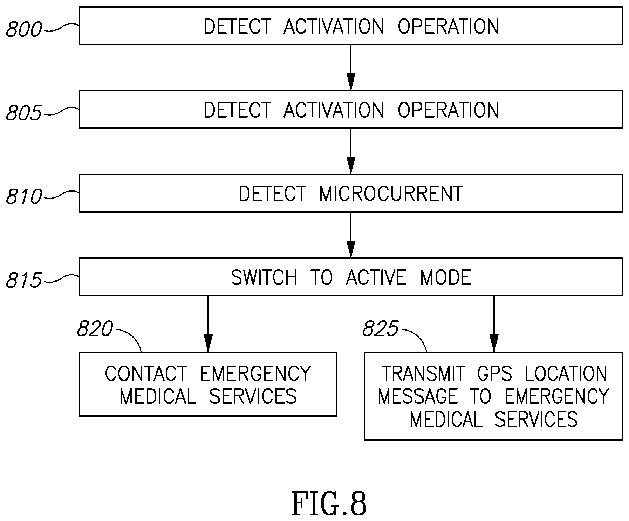

Fig. 8 outlines operations of a method for activating the resuscitation device 100 (Fig. 1), according to certain exemplary embodiments. In operation 800, processor 300 (Fig. 3) detects whether an activation operation has occurred. Resuscitation device 100 is configured to remain in a hibernation mode as long as case 200 (Fig. 2) remains sealed. Case sensor 325 (Fig. 3) detects that case 200 is opened and transmits a signal to processor 300.

In operation 805, processor 300 switches to a sleep mode. After processor 300 receives the detection that case 200 is opened, it switches from the hibernation mode to the sleep mode at which time. During sleep mode, processor 300 waits to receive a detection of microcurrent which signals to the processor 300 to switch to an active mode. In some embodiments, during sleep mode, processor 300 executes periodic internal diagnostic tests to ensure proper operation of resuscitation device 100 and operate electric pulse generator 320 (Fig. 3) to commence charging the electric charge that can be used to administer the electric pulse.

In operation 810, processor 300 detects a microcurrent. In some embodiments, the microcurrent can be detected by sensors 302, 304 (Fig. 3) when a cover (not shown) is removed from electrodes 120, 125 (Fig. 1), when electrodes 120, 125 are removed from a pack (not shown), when plastic line (not shown) is removed from electrodes 120, 125, or the like.

In operation 815, processor 300 switches to active mode. Upon detection of the microcurrent, processor 300 switches to active mode, which commences the operation of resuscitation device 100 in a configuration to administer the electric pulse, to monitor the vital signs of subject 185 (Fig. 1), communicate with mobile device 170 (Fig. 1), and the like.

In operation 820, processor 300 contacts emergency medical services.

In operation 825, processor transmits a GPS location message to emergency medical services.

In the context of some embodiments of the present disclosure, by way of example and without limiting, terms such as 'operating' or 'executing' also imply capabilities, such as 'operable' or 'executable', respectively.

Conjugated terms such as, by way of example, 'a thing property' implies a property of the thing, unless otherwise clearly evident from the context thereof.

The terms 'processor' or 'computer', or system thereof, are used herein as ordinary context of the art, such as a general purpose processor or a micro-processor, RISC processor, or DSP, possibly comprising additional elements such as memory or communication ports. Optionally or additionally, the terms 'processor' or 'computer' or derivatives thereof denote an apparatus that is capable of carrying out a provided or an incorporated program and/or is capable of controlling and/or accessing data storage apparatus and/or other apparatus such as input and output ports. The terms 'processor' or 'computer' denote also a plurality of processors or computers connected, and/or linked and/or otherwise communicating, possibly sharing one or more other resources such as a memory.

The terms 'software', 'program', 'software procedure' or 'procedure' or 'software code' or 'code' or 'application' may be used interchangeably according to the context thereof, and denote one or more instructions or directives or circuitry for performing a sequence of operations that generally represent an algorithm and/or other process or method. The program is stored in or on a medium such as RAM, ROM, or disk, or embedded in a circuitry accessible and executable by an apparatus such as a processor or other circuitry.

The processor and program may constitute the same apparatus, at least partially, such as an array of electronic gates, such as FPGA or ASIC, designed to perform a programmed sequence of operations, optionally comprising or linked with a processor or other circuitry.

The term computerized apparatus or a computerized system or a similar term denotes an apparatus comprising one or more processors operable or operating according to one or more programs.

As used herein, without limiting, a module represents a part of a system, such as a part of a program operating or interacting with one or more other parts on the same unit or on a different unit, or an electronic component or assembly for interacting with one or more other components.

As used herein, without limiting, a process represents a collection of operations for achieving a certain objective or an outcome.

As used herein, the term 'server' denotes a computerized apparatus providing data and/or operational service or services to one or more other apparatuses.

The term 'configuring' and/or 'adapting' for an objective, or a variation thereof, implies using at least a software and/or electronic circuit and/or auxiliary apparatus designed and/or implemented and/or operable or operative to achieve the objective.

A device storing and/or comprising a program and/or data constitutes an article of manufacture. Unless otherwise specified, the program and/or data are stored in or on a non-transitory medium.

In case electrical or electronic equipment is disclosed it is assumed that an appropriate power supply is used for the operation thereof.

The flowchart and block diagrams illustrate architecture, functionality or an operation of possible implementations of systems, methods and computer program products according to various embodiments of the present disclosed subject matter. In this regard, each block in the flowchart or block diagrams may represent a module, segment, or portion of program code, which includes one or more executable instructions for implementing the specified logical function(s). It should also be noted that, in some alternative implementations, illustrated or described operations may occur in a different order or in combination or as concurrent operations instead of sequential operations to achieve the same or equivalent effect.

The corresponding structures, materials, acts, and equivalents of all means or step plus function elements in the claims below are intended to include any structure, material, or act for performing the function in combination with other claimed elements as specifically claimed. As used herein, the singular forms "a", "an" and "the" are intended to include the plural forms as well, unless the context clearly indicates otherwise. It will be further understood that the terms "comprises" and/or "comprising" and/or "having" when used in this specification, specify the presence of stated features, integers, steps, operations, elements, and/or components, but do not preclude the presence or addition of one or more other features, integers, steps, operations, elements, components, and/or groups thereof.

As used herein the term "configuring" and/or 'adapting' for an objective, or a variation thereof, implies using materials and/or components in a manner designed for and/or implemented and/or operable or operative to achieve the objective.

Unless otherwise specified, the terms 'about' and/or 'close' with respect to a magnitude or a numerical value implies within an inclusive range of -10% to +10% of the respective magnitude or value.

Unless otherwise specified, the terms 'about' and/or 'close' with respect to a dimension or extent, such as length, implies within an inclusive range of -10% to +10% of the respective dimension or extent.

Unless otherwise specified, the terms 'about' or 'close' imply at or in a region of, or close to a location or a part of an object relative to other parts or regions of the object.

When a range of values is recited, it is merely for convenience or brevity and includes all the possible sub-ranges as well as individual numerical values within and about the boundary of that range. Any numeric value, unless otherwise specified, includes also practical close values enabling an embodiment or a method, and integral values do not exclude fractional values. A sub-range values and practical close values should be considered as specifically disclosed values. As used herein, ellipsis (...) between two entities or values denotes an inclusive range of entities or values, respectively. For example, A...Z implies all the letters from A to Z, inclusively.

The terminology used herein should not be understood as limiting, unless otherwise specified, and is for the purpose of describing particular embodiments only and is not intended to be limiting of the disclosed subject matter. While certain embodiments of the disclosed subject matter have been illustrated and described, it will be clear that the disclosure is not limited to the embodiments described herein. Numerous modifications, changes, variations and substitutions are not precluded as long as they fall within the scope of the appended claims. Terms in the claims that follow should be interpreted, without limiting, as characterized or described in the specification.

## Claims

1. A resuscitation device (100), comprising:
a housing (200);
an electric pulse generator (320) configured to generate an electric pulse that is administered to a subject;
electrodes (120, 125) operative to administer the electric pulse to the subject;
at least one sensor (302, 304) configured to measure vital signs of the subject;
at least one processing unit configured to:
monitor the vital signs measured by the at least one sensor (302, 304);
determine the housing (200) and electrodes (120, 125) are properly placed on the subject according to the monitoring of the vital signs;
determine what treatment has to be administered to the subject;
generate notification instructing the treatment to be administered to the subject; and,
providing real-time, continuous feedback of treatment provided and condition of the subject;
**characterized in that**
the at least one processing unit is configured to:
recognize that a mobile device (170) is within a predetermined distance;
receive authentication information from the mobile device (170);
determine the authentication information matches a stored authentication information;
grant the mobile communication access to communicate with the resuscitation device (100);
determining a plurality of mobile device have authority to access the resuscitation device (100);
allow access to the mobile device having a highest priority according to a priority hierarchy.

2. The resuscitation device according to claim 1, wherein said electric pulse generator (320) comprises an electrical circuit (400) for generating an electric pulse, said electric circuit comprises:
a controller (410);
a switch (430);
an inductor (420);
a charging circuit; and
a capacitor array (405) electrically connected to the charging circuit and adapted to store energy supplied by the charging circuit,
wherein the controller (410) is adapted to control the switch (430) to cause energy to be stored in the inductor (420) and discharged to the charging circuit.

3. The resuscitation device according to claim 2, wherein the controller (410) is a controller and gate driver chip, the inductor is a low-profile boost inductor (420), and the charging circuit is a plurality of interconnected charging capacitors (450) and diodes (460).

4. The resuscitation device according to claims 2-3, wherein the circuit comprises an electric pulse generator for a low profile AED.

5. The resuscitation device according to claims 2-4, wherein the capacitor array (405) comprises a plurality of interconnected discrete energy-storing capacitors capable of storing up to a predetermined amount of bi-phasic electric pulse energy.

6. The resuscitation device according to any of the preceding claims, further comprising:
a communication unit (305) configured to communicate with a mobile device (170), the mobile device (170) configured to:
present the user with user interface (325) and instructional content;
receive user commands for administering the electric pulse to the subject;
transmit the user commands to the communication unit (305).

7. The resuscitation device according to any of the preceding claims, further comprising:
a case;
an activation switch;
a case sensor configured to detect when the case is opened;
wherein said processor (300) is configured to:
switch from a sleep mode to a hibernation mode when said case sensor detects the case is opened;
switch to an active mode when the activation switch is engaged.

8. A system comprising:
a resuscitation device (100) according to claims 1-7;
at least one mobile device (170) configured to be in wireless communication with said resuscitation device (100), said at least one mobile device (170) configured to:
receive the vital signs;
present a user of the mobile device (170) with a user interface (325) configured to:
display the vital signs; and,
obtain commands from the user that are provided to the resuscitation device;
transmit the commands to the resuscitation device (100).

## Patentansprüche

1. Eine Wiederbelebungsvorrichtung (100), umfassend:
ein Gehäuse (200);
einen elektrischen Impulsgenerator (320), der so konfiguriert ist, dass er einen elektrischen Impuls erzeugt, der einem Patienten verabreicht wird;
Elektroden (120, 125), die dazu dienen, den elektrischen Impuls an den Patienten abzugeben;
mindestens einen Sensor (302, 304), der so konfiguriert ist, dass er die Vitalfunktionen des Patienten misst;
mindestens eine Verarbeitungseinheit, die dazu konfiguriert ist:
die von dem mindestens einen Sensor (302, 304) gemessenen Vitalparameter zu überwachen;
festzustellen, ob das Gehäuse (200) und die Elektroden (120, 125) entsprechend der Überwachung der Vitalparameter ordnungsgemäß an der Person angebracht sind;
zu bestimmen, welche Behandlung dem Patienten zu verabreichen ist;
eine Benachrichtigung zu generieren, die die dem Patienten zu verabreichende Behandlung anweist; und
Echtzeit-Feedback zur durchgeführten Behandlung und zum Zustand des Patienten bereitzustellen;
**dadurch gekennzeichnet, dass**
die mindestens eine Verarbeitungseinheit dazu konfiguriert ist:
zu erkennen, dass sich ein mobiles Gerät (170) innerhalb einer vorbestimmten Entfernung befindet;
Authentifizierungsinformationen von dem mobilen Gerät (170) zu empfangen;
festzustellen, ob die Authentifizierungsinformationen mit gespeicherten Authentifizierungsinformationen übereinstimmen;
dem mobilen Kommunikationsgerät den Zugriff für die Kommunikation mit dem Reanimationsgerät (100) zu gewähren;
festzustellen, dass mehrere mobile Geräte berechtigt sind, auf das Wiederbelebungsgerät (100) zuzugreifen;
dem Mobilgerät mit der höchsten Priorität gemäß einer Prioritätshierarchie den Zugriff zu gewähren.

2. Die Wiederbelebungsvorrichtung gemäß Anspruch 1, wobei der elektrische Impulsgenerator (320) eine elektrische Schaltung (400) zum Erzeugen eines elektrischen Impulses umfasst, wobei die elektrische Schaltung umfasst:
eine Kontrolleinheit (410);
einen Schalter (430);
eine Induktivität (420);
eine Ladeschaltung; und
eine Kondensatoranordnung (405), die elektrisch mit der Ladeschaltung verbunden ist und dazu ausgelegt ist, von der Ladeschaltung gelieferte Energie zu speichern,
wobei die Kontrolleinheit (410) dazu ausgelegt ist, den Schalter (430) so zu steuern, dass Energie in der Induktivität (420) gespeichert und an die Ladeschaltung abgegeben wird.

3. Die Wiederbelebungsvorrichtung nach Anspruch 2, wobei die Kontrolleinheit (410) ein Steuer- und Gate-Treiber-Chip ist, die Induktivität eine flache Boost-Induktivität (420) ist und die Ladeschaltung eine Vielzahl miteinander verbundener Ladekondensatoren (450) und Dioden (460) ist.

4. Die Wiederbelebungsvorrichtung nach den Ansprüchen 2 bis 3, wobei die Schaltung einen elektrischen Impulsgenerator für einen flachen AED umfasst.

5. Die Wiederbelebungsvorrichtung nach den Ansprüchen 2 bis 4, wobei die Kondensatoranordnung (405) eine Vielzahl miteinander verbundener diskreter Energiespeicherkondensatoren umfasst, die in der Lage sind, bis zu einer vorbestimmten Menge an biphasischer elektrischer Impulsenergie zu speichern.

6. Die Wiederbelebungsvorrichtung nach einem der vorstehenden Ansprüche, das ferner umfasst:
eine Kommunikationseinheit (305), die für die Kommunikation mit einem mobilen Gerät (170) konfiguriert ist, wobei das mobile Gerät (170) dazu konfiguriert ist:
dem Benutzer eine Benutzeroberfläche (325) und Anweisungsinhalte zu präsentieren;
Benutzerbefehle zur Verabreichung des elektrischen Impulses an den Patienten zu empfangen;
die Benutzerbefehle an die Kommunikationseinheit (305) zu übertragen.

7. Die Wiederbelebungsvorrichtung gemäß einem der vorstehenden Ansprüche, das ferner umfasst:
ein Gehäuse;
einen Aktivierungsschalter;
einen Gehäusesensor, der so konfiguriert ist, dass er erkennt, wenn das Gehäuse geöffnet wird;
wobei der Prozessor (300) dazu konfiguriert ist:
von einem Schlafmodus in einen Ruhemodus zu wechseln, wenn der Gehäusesensor erkennt, dass das Gehäuse geöffnet ist;
in einen aktiven Modus zu wechseln, wenn der Aktivierungsschalter betätigt wird.

8. Ein System, umfassend:
eine Wiederbelebungsvorrichtung (100) gemäß einem der Ansprüche 1 bis 7;
mindestens ein mobiles Gerät (170), das so konfiguriert ist, dass es mit der Wiederbelebungsvorrichtung (100) drahtlos kommuniziert, wobei das mindestens eine mobile Gerät (170) dazu konfiguriert ist:
Vitalparameter zu empfangen;
einem Benutzer des mobilen Geräts (170) eine Benutzeroberfläche (325) zu präsentieren, die dazu konfiguriert ist:
die Vitalzeichen anzuzeigen; und
Befehle vom Benutzer zu erhalten, die an das Wiederbelebungsgerät weitergeleitet werden;
die Befehle an das Wiederbelebungsgerät (100) zu übertragen.

## Revendications

1. Dispositif de réanimation (100), comprenant :
un boîtier (200) ;
un générateur d'impulsions électriques (320) configuré pour générer une impulsion électrique qui est administrée à un sujet ;
des électrodes (120, 125) fonctionnant pour administrer l'impulsion électrique au sujet ;
au moins un capteur (302, 304) configuré pour mesurer les signes vitaux du sujet ;
au moins une unité de traitement configurée pour :
surveiller les signes vitaux mesurés par le au moins un capteur (302, 304) ;
déterminer si le boîtier (200) et les électrodes (120, 125) sont correctement placés sur le sujet en fonction de la surveillance des signes vitaux ;
déterminer le traitement à administrer au sujet ;
générer une notification indiquant le traitement à administrer au sujet ; et
fournir en temps réel et en continu des informations sur le traitement administré et l'état du sujet ;
**caractérisé en ce que**
la au moins une unité de traitement est configurée pour :
reconnaître qu'un dispositif mobile (170) se trouve à une distance prédéterminée ;
recevoir des informations d'authentification provenant du dispositif mobile (170) ;
déterminer si les informations d'authentification correspondent à des informations d'authentification stockées ;
accorder à la communication mobile l'accès pour communiquer avec le dispositif de réanimation (100) ;
déterminer qu'une pluralité d'appareils mobiles ont l'autorisation d'accéder à l'appareil de réanimation (100) ;
autoriser l'accès à l'appareil mobile ayant la priorité la plus élevée selon une hiérarchie de priorités.

2. Le dispositif de réanimation selon la revendication 1, dans lequel ledit générateur d'impulsions électriques (320) comprend un circuit électrique (400) pour générer une impulsion électrique, ledit circuit électrique comprenant :
un contrôleur (410) ;
un commutateur (430) ;
une inductance (420) ;
un circuit de charge ; et
un réseau de condensateurs (405) connecté électriquement au circuit de charge et adapté pour stocker l'énergie fournie par le circuit de charge,
dans lequel le contrôleur (410) est adapté pour commander le commutateur (430) afin de provoquer le stockage d'énergie dans l'inductance (420) et sa décharge vers le circuit de charge.

3. Dispositif de réanimation selon la revendication 2, dans lequel le contrôleur (410) est une puce de contrôleur et de commande de porte, l'inductance est une inductance élévatrice à profil bas (420) et le circuit de charge est constitué d'une pluralité de condensateurs de charge (450) et de diodes (460) interconnectés.

4. Dispositif de réanimation selon les revendications 2 à 3, dans lequel le circuit comprend un générateur d'impulsions électriques pour un DAE à profil bas.

5. Dispositif de réanimation selon les revendications 2 à 4, dans lequel le réseau de condensateurs (405) comprend une pluralité de condensateurs discrets interconnectés capables de stocker jusqu'à une quantité prédéterminée d'énergie d'impulsion électrique biphasique.

6. Dispositif de réanimation selon l'une quelconque des revendications précédentes, comprenant en outre :
une unité de communication (305) configurée pour communiquer avec un dispositif mobile (170), le dispositif mobile (170) étant configuré pour :
présenter à l'utilisateur une interface utilisateur (325) et un contenu instructif recevoir les commandes de l'utilisateur pour administrer l'impulsion électrique au sujet ;
transmettre les commandes de l'utilisateur à l'unité de communication (305).

7. Dispositif de réanimation selon l'une quelconque des revendications précédentes, comprenant en outre :
un boîtier ;
un commutateur d'activation ;
un capteur de boîtier configuré pour détecter lorsque le boîtier est ouvert ;
dans lequel ledit processeur (300) est configuré pour :
passer d'un mode veille à un mode veille prolongée lorsque ledit capteur de boîtier détecte que le boîtier est ouvert ;
passer en mode actif lorsque le commutateur d'activation est enclenché.

8. Système comprenant :
un dispositif de réanimation (100) selon les revendications 1 à 7 ;
au moins un dispositif mobile (170) configuré pour être en communication sans fil avec ledit dispositif de réanimation (100), ledit au moins un dispositif mobile (170) étant configuré pour :
recevoir les signes vitaux ;
présenter à un utilisateur du dispositif mobile (170) une interface utilisateur (325) configurée pour :
afficher les signes vitaux ; et
obtenir des commandes de l'utilisateur qui sont fournies au dispositif de réanimation ;
transmettre les commandes au dispositif de réanimation (100).
